# EUROPÄISCHE PATENTSCHRIFT

(11) **EP 1 656 905 B1**
(45) Veröffentlichungstag und Bekanntmachung des Hinweises auf die Patenterteilung: **27.12.2006**
(21) Anmeldenummer: 05019459.6
(22) Anmeldetag: 07.09.2005
(51) Int. Cl.: A61F 2/06

(54) **Verfahren zum Anbringen von röntgensichtbaren Markern an einen Stent**
Method for attaching radiopaque markers to a stent
Procédé pour attacher des marqueurs radio-opaques au stent

(30) Priorität: 09.11.2004 DE 102004054084
(43) Veröffentlichungstag der Anmeldung: 17.05.2006
(73) Patentinhaber: Admedes Schuessler GmbH, 75179 Pforzheim (DE)
(72) Erfinder: Flaxmeier, Erik, 76307 Karlsbad (DE); Mailänder, Werner, 75217 Birkenfeld (DE)
(74) Vertreter: Müller-Boré & Partner Patentanwälte

(56) Entgegenhaltungen:
- WO-A-02/26162
- WO-A-03/015664
- DE-A1- 10 064 596
- DE-U1-9202004 014 78
- US-A1- 2004 088 039
- US-B1- 6 293 966

## Beschreibung

Die vorliegende Erfindung betrifft einen Stent mit einem Marker zur Verbesserung der Röntgensichtbarkeit des Stents sowie ein Verfahren zum Herstellen eines derartigen Stents.

Stents werden verwendet, um verschieden Kanäle lebender Körper, wie z.B. Blutgefäße, Speiseröhre, Harnröhre, Nierengänge, durch Expandieren einer röhrenförmigen Stentstruktur im Inneren des Kanals gegen Kollabieren oder Verschließen zu schützen und/oder als Träger von Medikamenten in Körperkanälen eine zumindest lokale Therapie zu ermöglichen. Stents können darüber hinaus als Aneurismen-Stents bzw. Endoprothese für intrazelebrale Gefäßaussackungen oder als intraluminaler Stent eingesetzt werden.

Der Stent muß radial im Kanal expandierbar sein, um die Kanalwand zu stützen. Darüber hinaus muß der Stent im expandierten Zustand flexibel bzw. schlauchartig sein, um die Stützfunktion auch in gebogenen Kanal- bzw. Aderbereichen zu ermöglichen. Weiterhin muß der Stent auch im komprimierten Zustand flexibel sein, um durch gebogene bzw. kurvige Kanäle und Blutgefäße gelangen zu können.

Um dies zu erreichen werden bei bekannten Stents verschiedene funktionale Geometrieelemente als Wandabschnitte zu einer Vielzahl Zellen kombiniert, die aneinander angrenzen. Die einzelnen Wandabschnitte sind in bestimmter Weise, beispielsweise gekrümmt oder gerade, ausgebildet, um dem Stent die gewünschten Deformationseigenschaften zu verleihen. Damit eine radiale Expansion des Stents ermöglicht ist, sind sogenannten Zick-Zack-Strukturen ausgebildet, die durch Brücken verbunden sind. Die Brücken können sich beim Strecken der Zick-Zack-Strukturen verformen bzw. abflachen und ermöglichen so eine tangentenähnliche Biegelinie des Stents.

Stents werden in einen lebenden Körper eingeführt, um ein Gefäß zu stützen oder zu erweitern. Hierzu werden diese Stents in einen Katheter geladen und in das Gefäß eingeführt. Nach der Plazierung in dem Gefäß wird der Stent durch seine Formgedächtniseigenschaften oder mit Hilfe eines Ballonkatheders erweitert. Als Stentmaterial mit geeigneten Formgedächtniseigenschaften eignet sich insbesondere Nitinol (Nickel-Titan-Legierung). Für ballonexpandierbare Stents eignen sich insbesondere Edelstahl und Kobalt-Chrom. Jedoch sind diese Materialien bei einer Röntgenuntersuchung ungenügend sichtbar.

Deshalb muß ein aus Nitinol, Edelstahl oder Cobalt-Chrom bestehender Stent mit Markern zur Verbesserung der Röntgensichtbarkeit versehen werden.

US 6,355,058 offenbart hierzu einen Stent mit einer röntgensichtbaren Beschichtung. WO 02/15820 offenbart einen Stent mit Tantalmarkern, die durch Laserschweißen an der Stentstruktur angebracht werden. US 2002/01 93 867 offenbart einen Stent, bei dem ein Markereinsatz aus Tantal in einem Markergehäuse der Stentstruktur eingebettet ist. Schließlich offenbart US 6,635,082 eine Stentstruktur mit Mikrodepots in einer Kegelstumpfform, in die ein röntgensichtbares Metall eingebracht wird, das in einer Polymerlösung suspendiert ist.

WO 02/26162 A2 offenbart einen Stent mit einem röntgensichtbaren Marker, der in einer Ausnehmung der Stentstruktur aufgenommen ist und wobei ein Restvolumen der Ausnehmung mit einem Polymer aufgefüllt ist.

DE 100 64 596 A1 beschreibt ein Implantat mit einem Markerelement, das in einer Ausnehmung des Implantats aufgenommen ist, wobei ein verfestigbares Material in die Ausnehmung eingebracht und dort verfestigt wird.

US 6 293 966 B1 offenbart einen Stent mit röntgensichtbaren Markern, wobei der Stent an seinen Enden Aufnahmen aufweist, an denen die röntgensichtbaren Markerelemente angebracht werden können.

Die Aufgabe der Erfindung besteht in der Schaffung eines Stents mit einer verbesserten Röntgensichtbarkeit sowie eines Herstellverfahrens eines derartigen Stents.

Diese Aufgabe wird durch einen Stent nach Anspruch 1 und ein Verfahren nach Anspruch 9 gelöst. Vorteilhafte Weiterbildungen der Erfindung sind in den abhängigen Ansprüchen angeführt.

Indem ein röntgensichtbarer Marker aus einem Bandmaterial bzw. einem Draht an dem Stent angebracht wird, ist die Röntgensichtbarkeit verbessert. Indem des weiteren ein Restvolumen der Ausnehmung der Stentstruktur mit einem Polymer aufgefüllt wird, wird der Marker auf einfache Weise an der Stentstruktur fixiert. Der Marker kann dabei aus einem Bandmaterial mit rechteckigem Querschnitt oder einem Bandmaterial mit kreisrundem Querschnitt bzw. einem Draht gebildet werden. Dieser Draht bzw. das Band hat eine hohe Röntgensichtbarkeit.

Vorzugsweise hat der Marker einen kreisrunden Querschnitt und die Ausnehmung der Stentstruktur eine Keilform, so dass der Marker sich in der Ausnehmung selbsttätig zentriert.

Indem der Marker einen kreisrunden Querschnitt aufweist und die Ausnehmung der Struktur eine Keilform aufweist, kann sich der Marker aufgrund seiner Schwerkraft selbsttätig in der Ausnehmung an den Schrägen der Keilform zentrieren. Eine Justierung des Markers in der Ausnehmung ist deshalb nicht nötig.

Vorzugsweise mündet die keilförmige Ausnehmung in eine Öffnung, die einen Durchlass nach einer entgegengesetzten Seite der Stentstruktur aufweist.

Indem die keilförmige Ausnehmung in eine Öffnung mündet mit einem Durchlaß nach einer entgegengesetzten Seite der Stentstruktur, kann überschüssiges Polymer beim Vergießen des Markers abfließen.

Vorzugsweise hat die Ausnehmung eine im wesentlichen gleichmäßige Breite sowie einen Bereich mit verringerter Breite zum Klemmen des Markers.

Vorzugsweise hat entweder die Ausnehmung oder der Marker einen Vorsprung und das andere Element aus der Ausnehmung und dem Marker eine Vertiefung für eine Schnappverbindung des Markers in der Ausnehmung.

Die Vorsprünge sind vorzugweise wellenförmig angeordnet.

Indem der Marker in der Ausnehmung geklemmt wird bzw. über eine Schnappverbindung mit dieser in Eingriff tritt, wird der Marker selbsttätig in der Ausnehmung justiert. Ein Schritt des Justierens des Markers in der Ausnehmung bei der Herstellung des Stents kann somit entfallen. Hierdurch können die Herstellkosten aufgrund der vereinfachten Fertigung reduziert werden.

Des weiteren schafft die vorliegende Erfindung ein Verfahren zur Verbesserung der Röntgensichtbarkeit eines Stents mit den Schritten nach Anspruch 9.

Die Erfindung wird unter Bezugnahme auf die Zeichnungen nachfolgend näher erläutert.
Fig. 1 zeigt einen Schnitt durch eine Stentstruktur mit einem darin aufgenommenen Marker gemäß einem ersten Ausführungsbeispiel.
Fig. 2 zeigt einen Schnitt durch eine erfindungsgemäße Stentstruktur mit einem darin aufgenommenen Marker gemäß einem zweiten Ausführungsbeispiel.
Fig. 3 zeigt einen Schnitt durch eine erfindungsgemäße Stentstruktur mit einem darin aufgenommenen Marker gemäß einem dritten Ausführungsbeispiel.
Fig. 4 zeigt einen Schnitt durch eine erfindungsgemäße Stentstruktur mit einem darin aufgenommenen Marker gemäß einem vierten Ausführungsbeispiel.
Fig. 5 zeigt einen Schnitt durch eine erfindungsgemäße Stentstruktur mit einem darin aufgenommenen Marker gemäß einem fünften Ausführungsbeispiel.
Fig. 6 zeigt eine erfindungsgemäße Stentstruktur mit an unterschiedlichen Stellen der Stentstruktur angeordneten Markern.
Fig. 7a bis c zeigen eine Ansicht A-A von Fig. 2 von unterschiedlichen. Stentstrukturen zur Aufnahme eines Markers.

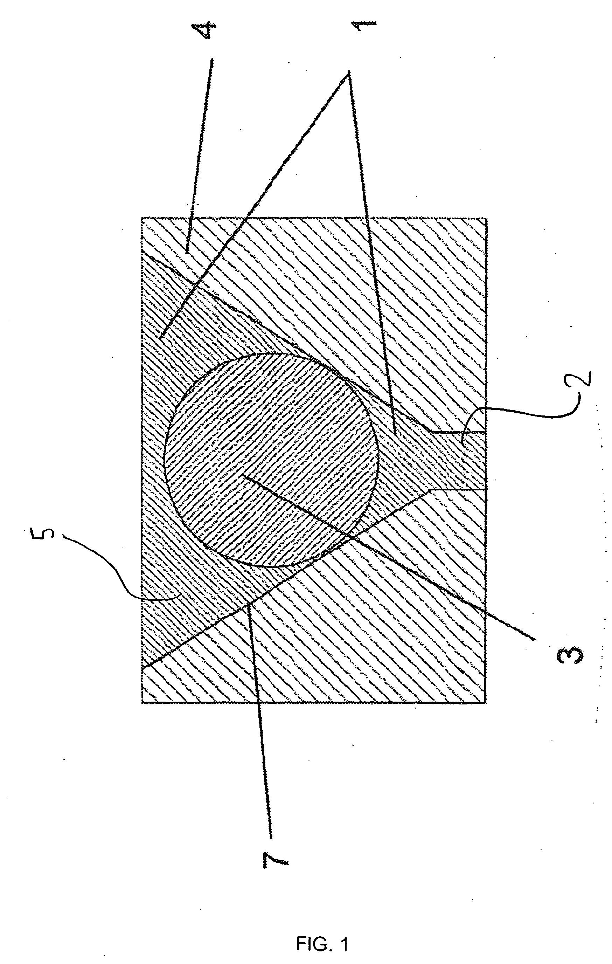

Die Erfindung wird nachfolgend anhand bevorzugter Ausführungsbeispiele näher erläutert. Fig. 1 zeigt den Schnitt durch eine Stentstruktur mit einem darin eingelagerten Marker 3 gemäß einem ersten Ausführungsbeispiel. Es ist eine keilförmige Ausnehmung mit einer Schräge 7 in einer Stentwand 4 ausgeformt. Darüber hinaus mündet die keilförmige Ausnehmung 5 in einer Öffnung 2, die einen Durchlaß zu einer entgegengesetzten Seite der Stentstruktur bildet.

Die Ausnehmung 5 sowie die Öffnung 2 können beispielsweise durch eine Laserbearbeitung der Stentstruktur gebildet werden. Vorzugsweise wird dabei eine gekühlte Flüssigkeit oder ein Fluid durch den rohrförmigen Stent hindurchgeleitet, so dass ein durch die Rohrwandung des Stents hindurchtretender Laserstrahl im Inneren des Rohrs refraktiert wird. Es kann jedoch auch ein mechanisches Bearbeitungsverfahren, wie Fräsen, Bohren und Schleifen oder Erodieren zum Einsatz kommen.

In diese keilförmige Ausnehmung 5 kann eine geeignetes Bandmaterial als ein röntgensichtbarer Marker 3 eingelegt werden. Dieses Bandmaterial hat einen rechteckige oder eine kreisrunde Querschnittsform. Jedoch ist der Querschnitt des Bandmaterials nicht hierauf beschränkt. Insbesondere kann es auch eine Elipsenform oder eine Dreiecksform, oder andere Polygonform haben. Wie in Fig. 1 gezeigt ist, liegt der bandartige Marker 3 an den Schrägen 7 der Keilform der Ausnehmung 5 an. Hierdurch zentriert sich der Marker 3 selbsttätig, so daß eine Justierung des Markers 3 innerhalb der Ausnehmung 5 überflüssig ist. Der Marker 3 besteht beispielsweise aus Tantal oder einem anderen röntgensichtbaren Material.

Nach dem Einlegen des Markers 3 in die Ausnehmung 5 wird ein Restvolumen der Ausnehmung 5 mit einem Polymer 1 gefüllt, wodurch der Marker 3 in der Ausnehmung 5 fixiert wird. Überschüssiges Polymer 1 kann dabei über die Öffnung 2 zu der entgegengesetzten Seite der Stentstruktur abfließen.

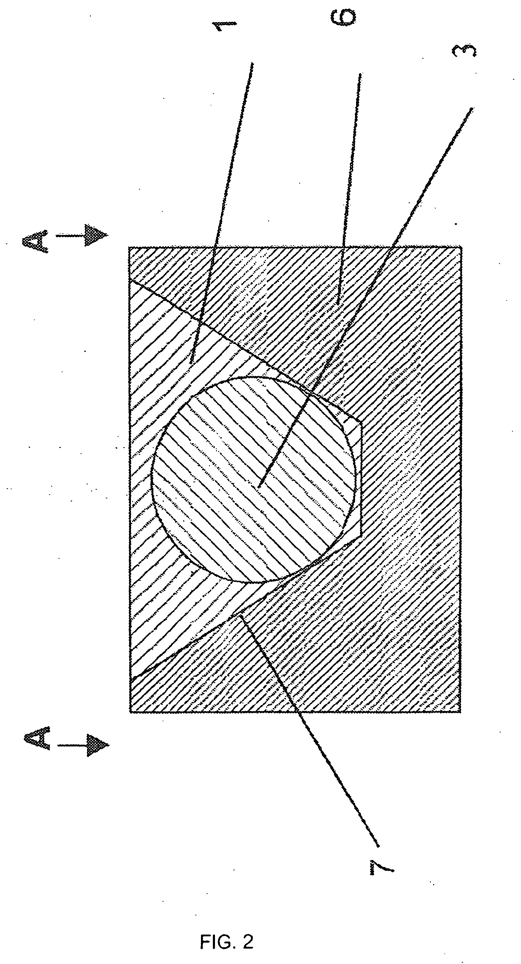

Fig. 2 zeigt ein zweites Ausführungsbeispiel ähnlich dem ersten Ausführungsbeispiel, das in Fig. 1 gezeigt ist. Gleiche Merkmale des zweiten Ausführungsbeispiels werden deshalb nicht nochmals erläutert. Der Unterschied des zweiten Ausführungsbeispiels zu dem ersten Ausführungsbeispiel besteht darin, daß die Öffnung 2 des ersten Ausführungsbeispiels in dem zweiten Ausführungsbeispiel von Fig. 2 nicht ausgebildet ist. Hierdurch ergibt sich der Vorteil, daß das Polymermaterial 1 nur von einer Seite eingebracht werden muß.

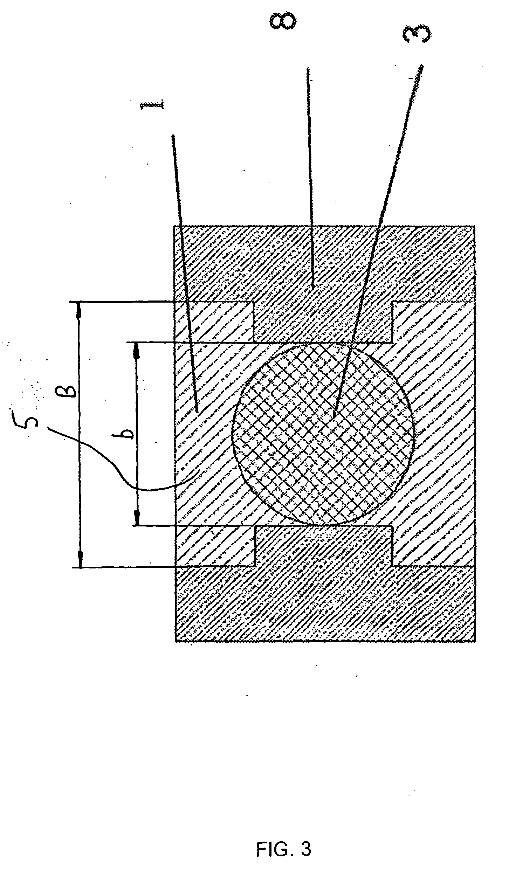

Unter Bezugnahme auf Fig. 3 wird ein drittes Ausführungsbeispiel der vorliegenden Erfindung erläutert. Bei diesem Ausführungsbeispiel hat die Ausnehmung 5 im wesentlichen eine rechteckige Form mit gleichmäßiger Breite B. Darüber hinaus ist die Ausnehmung 5 in einem zentralen Bereich in ihrer Breite auf eine geringere Breite b verringert. Die Breite b ist dabei etwas geringer als die Dicke des Bandmaterials des Markers 3, so daß der Marker 3 in der Ausnehmung 5 festgeklemmt werden kann. Hierdurch erfolgt wiederum eine selbsttätige Zentrierung des Markers innerhalb der Stentstruktur. Der Marker 3 muß hierbei keine kreisförmige Querschnittsform haben, sondern kann auch eine rechteckige oder quadratische oder beliebige andere Form haben, solange wie die Seiten des Markers 3, die mit den Wänden 8 der Stentstruktur in Eingriff treten, etwas abgerundet sind, um mit der Ausnehmung 5 an der verengten Stelle mit der Breite b im Klemmsitz in Eingriff zu treten. Der Vorteil dieses Ausführungsbeispiels liegt in einer gleichmäßig wirkenden Fixierkraft des Polymers 1 sowohl in einer Aufwärts- als auch in einer Abwärtsrichtung. Da diese Ausführungsform zu einer symmetrischen Querschnittsform der Stentstruktur führt, ist die Röntgensichtbarkeit besonders vorteilhaft ausgeprägt.

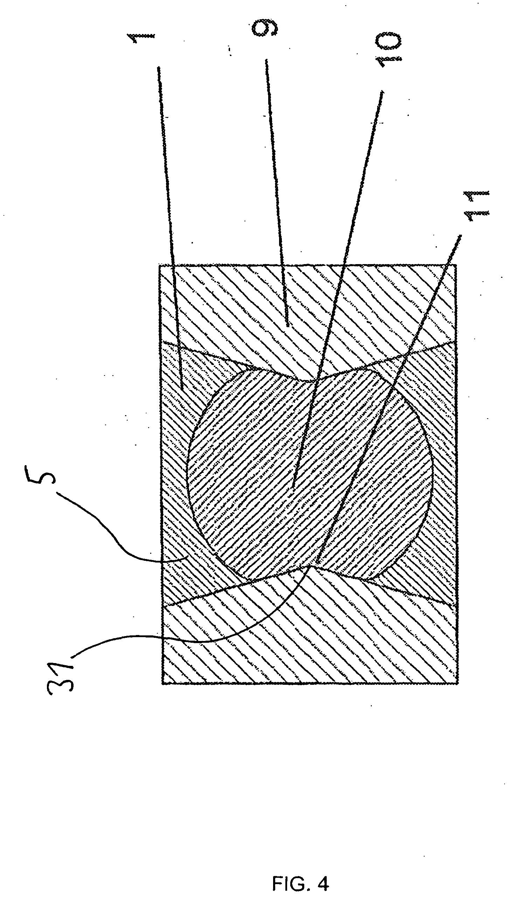

Unter Bezugnahme auf Fig. 4 wird ein viertes bevorzugtes Ausführungsbeispiel erläutert. Bei diesem Ausführungsbeispiel weist die Ausnehmung 5 in einem zentralen Bereich einen Vorsprung 31 auf und der Marker 10 ist mit einer Vertiefung 11 versehen. Die Form des Vorsprungs 31 der Ausnehmung 5 ist so an die Form der Vertiefung 11 des Markers 10 angepaßt, daß der Marker 10 in den Vorsprung 31 der Ausnehmung 5 einschnappen kann. Hierdurch wird der Marker 10 bereits in der Ausnehmung 5 der Stentstruktur fixiert. Die endgültige Fixierung erfolgt dann wiederum durch Einbringen eines Polymermaterials 1. Durch das Einschnappen des Markers 10 in der Ausnehmung 5 erfolgt eine Vor-Fixierung, so daß eine Fixierung des Markers 10 während dem Vergießen mit dem Polymermaterial 1 nicht notwendig ist. Hierdurch kann die Herstellung des Stents mit dem darin fixierten Marker 10 vereinfacht werden. Da der Querschnitt der Stentstruktur des vierten Ausführungsbeispiels darüber hinaus eine symmetrische Form hat, ist die Röntgensichtbarkeit besonders vorteilhaft.

Die Vor-Fixierung des vierten Ausführungsbeispiels ist jedoch nicht auf die Ausbildung des Vorsprungs 31 und die Vertiefung 11 beschränkt. Es kann auch jede andere Einschnappverbindung zwischen dem Bandmaterial des Markers 10 und der Ausnehmung 5 der Stentstruktur zur Anwendung kommen. Beispielsweise kann das Bandmaterial 10 einen Vorsprung haben und die Ausnehmung 5 eine Vertiefung.

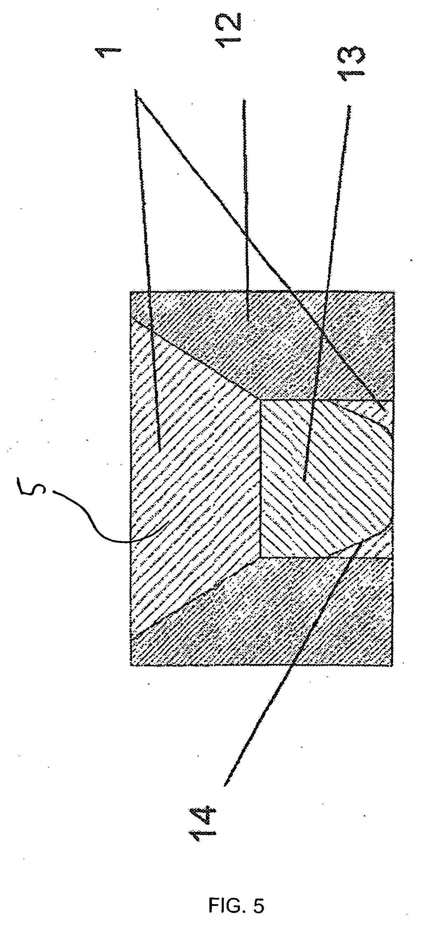

Unter Bezugnahme auf Fig. 5 wird ein fünftes Ausführungsbeispiel der vorliegenden Erfindung erläutert. Bei diesem Ausführungsbeispiel wird ein Bandmaterial mit einem im wesentlichen quadratischen Querschnitt als ein Marker 13 verwendet. Der quadratische Querschnitt des Markers 13 ist jedoch in einer Einsetzrichtung des Markers 13 mit einer Schräge 14 zum Erleichtern des Einsetzens des Markers 13 versehen. Darüber hinaus ist die Ausnehmung 5 der Stentstruktur in einem oberen Bereich keilförmig ausgebildet und in einem unteren Bereich mit gleichmäßiger Breite ausgebildet. Die Breite der Ausnehmung 5 ist dabei vorzugsweise geringfügig kleiner dimensioniert als die Breite des Markers 13, so daß eine Klemmverbindung zwischem dem Marker 13 und der Ausnehmung 5 der Stentstruktur zustande kommt. Ein Restvolumen der Ausnehmung 5 wird wiederum- durch ein Polymermaterial 1 vergossen.

Als ein Polymermaterial für alle bisher beschriebenen Ausführungsbeispiele 1 bis 5 eignet sich insbesondere Polytetrafluorethylen, Fluorethylenpropylen, Polyvinylfluorid, Polyetherurethan, Polyesterurethan, Polycarbonaturethan, Silikon, Polyphosphazene, Polyphosphorester, Polyactide, Polyanhydride, Polyimid, Polyethylen, Polypropylen, Ethylenvinylacetat, Polyetherketone, Polyaryletherketone oder Polysulfone. Als das Material für den Marker 3, 10, 13 eignet sich insbesondere Wolfram, -Tantal, Gold, Platin, Niob, Palladium, Silber, Iridium sowie Legierungen hiervon.

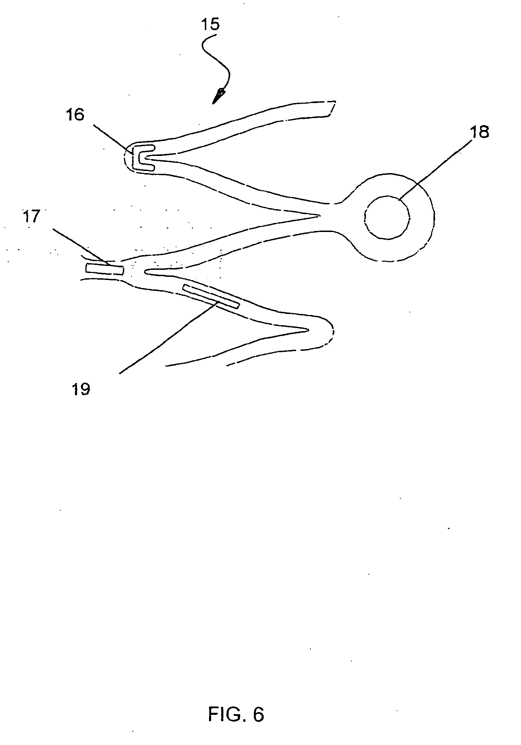

Die Ausnehmungen 5 innerhalb der Stentstruktur können an verschiedenen Stellen eines Stents angebracht werden. Wie in Fig. 6 gezeigt ist, können die Ausnehmungen 5 an einem Scheitelpunkt 16, einem Steg oder Zellenverbinder 17 oder an einem Steg 19 einer Stentstruktur angebracht werden. Darüber hinaus können die Ausnehmungen 5 an Stentenden 18 angebracht werden.

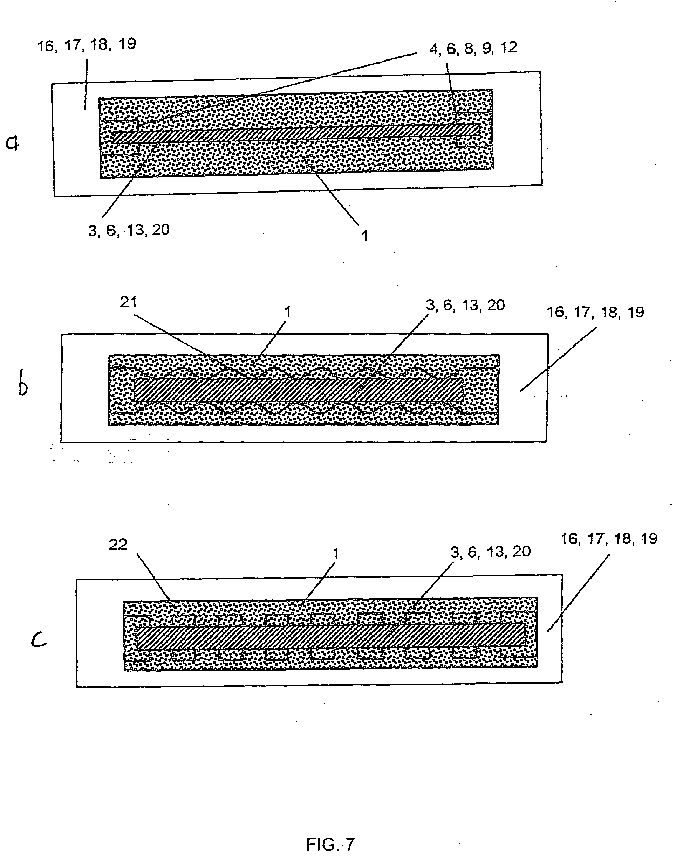

Fig. 7 zeigt eine Ansicht A-A von Fig. 2. Gemäß Fig. 7a bis c hat die Ausnehmung 5 in der Längsrichtung unterschiedliche Formen. Wie in Fig. 7a gezeigt ist, kann die Ausnehmung 5 an ihren Enden einen Bereich mit vergrößerter Breite aufweisen. Wie in Fig. 7b gezeigt ist, kann die Breite der Ausnehmung 5 wellenförmig variieren, so daß der Marker 3, 6, 13, 20 an den Wellenerhebungen der Ausnehmung 5 geklemmt wird. Wie in Fig. 7c gezeigt ist, kann die Ausnehmung 5 ein Sägezahnprofil haben, so daß der Marker 3, 6, 13, 20 mit den Zähnen der Ausnehmung geklemmt wird. Die Erfindung ist jedoch nicht auf die hier gezeigten Beispiele beschränkt. Die Ausnehmung 5 kann jede andere Form haben. Der Marker sollte lediglich an voneinander beabstandeten Stellen in der Längsrichtung der Stentstruktur durch Vorsprünge der Ausnehmung geklemmt werden, so daß eine Vor-Fixierung vor dem Vergießen mit dem Polymer 1 erzielt werden kann. Hierzu eignet sich insbesondere auch ein Zick-Zack-Profil.

Der Einsatz eines Bandmaterials bietet den Vorteil, dass nicht nur einzelne Stellen wie Enden der Stentstruktur durch den Marker sichtbar gemacht werden können, sondern die gesamte Stentstruktur sichtbar gemacht werden kann, wenn sich das Bandmaterial über eine größere Länge der Stege einer Stentstruktur erstreckt.

Die Erfindung ist auch nicht auf Stents aus Nitinol beschränkt, sondern kann auf Stents aus anderen Materialien angewandt werden. Beispiels hierfür sind Stents aus Edelstahl oder Kobalt-Chrom. Die Erfindung bietet insbesondere den Vorteil, daß das Bandmaterial für die Ausbildung des Markers gegenüber einem Marker aus einem Polymer mit röntgensichtbarem Pulver eine erhöhte verbesserte Röntgensichtbarkeit aufweist. Gegenüber dem Anbringen eines Markers mittels Laserschweißen und Beschichten der Stentstruktur bietet die vorliegende Erfindung ein vereinfachtes Verfahren zum Anbringen eines Markers an einer Stentstruktur.

Der Marker ist jedoch nicht auf das hier beschriebene Bandmaterial beschränkt. Der Marker kann jede beliebige andere Form haben, beispielsweise die Form einer Kugel, eines Stifts oder eines Kegels. Es kommt lediglich darauf an, dass ein Festkörper aus einem röntgensichtbaren Material in einer Ausnehmung der Stentstruktur plaziert wird und mit einem Polymer vergossen wird.

Vorteilhaft ist dabei ein Klemmsitz oder eine Schnappverbindung des Festkörpers innerhalb der Ausnehmung. Hierzu kann die Ausnehmung jede beliebige zu der Festkörperform passende Form haben, beispielsweise eine zylindrische Form, eine konische Form oder eine Form mit Vorsprüngen und/oder Vertiefungen zum Bilden einer Schnappverbindung.

### Bezugszeichenliste

- 1: Polymer
- 2: Öffnung
- 3: Marker
- 4: Stentwand
- 5-: Ausnehmung
- 6: Stentwand
- 7: Schräge
- 8: Stentwand
- 9: Stentwand
- 10: Marker
- 11: Vertiefung
- 12: Stentwand
- 13: Marker
- 14: Schräge
- 15: Stentstruktur
- 16: Marker
- 17: Marker
- 18: Marker
- 19: Marker
- 20: Marker
- 31: Vorsprung

## Patentansprüche

1. Stent zum Implantieren in einen lebenden Körper mit einem röntgensichtbaren Marker (3, 10, 13), der in einer Ausnehmung (16, 17, 18, 19) der Stentstruktur aufgenommen ist und wobei ein Restvolumen der Ausnehmung mit einem Polymer (1) aufgefüllt ist,
**dadurch gekennzeichnet, dass**
der Marker stangenförmig ausgebildet ist und Seitenwände der Ausnehmung in einer Längsrichtung eine Vielzahl an Vorsprüngen zum Klemmen des Markers aufweisen.

2. Stent nach Anspruch 1, wobei der Marker (3) einen kreisrunden Querschnitt aufweist und die Ausnehmung der Stentstruktur eine Keilform aufweist, um beim Einlegen des Markers in die Ausnehmung das selbsttätige Zentrieren des Markers zu ermöglichen.

3. Stent nach Anspruch 2, wobei die keilförmige Ausnehmung in eine Öffnung mündet, die einen Durchlass nach einer entgegengesetzten Seite der Stentstruktur aufweist.

4. Stent nach Anspruch 1, wobei die Ausnehmung eine im wesentlichen gleichmäßige Breite (B) aufweist.

5. Stent nach Anspruch 4, wobei die Ausnehmung einen Bereich mit verringerter Breite (b) zum Klemmen des Markers (3) aufweist.

6. Stent nach Anspruch 4 oder 5, wobei der Marker (3) eine Vertiefung aufweist für eine Schnappverbindung des Markers (3) in der Ausnehmung.

7. Stent nach einem der vorherigen Ansprüche, wobei die Vorsprünge wellenförmig angeordnet sind.

8. Stent nach einem der Ansprüche 1 bis 6, wobei die Vorsprünge als Zick-Zack-Profil angeordnet sind.

9. Verfahren zur Verbesserung der Röntgensichtbarkeit eines Stents mit den Schritten:
Bilden einer Ausnehmung in der Stentstruktur;
Einsetzen eines Markers in die Ausnehmung; und
Auffüllen der Ausnehmung mit einem Polymer;
**gekennzeichnet durch**
Ausbilden des Markers in einer Stangenform; und
Ausbilden einer Vielzahl von Vorsprüngen in Seitenwänden der Ausnehmung in einer Längsrichtung zum Klemmen des Markers.

## Claims

1. Stent for implantation in a living body, with a radiopaque marker (3, 10, 13) which is received in a recess (16, 17, 18, 19) of the stent structure, a residual volume of the recess being filled with a polymer (1),
**characterized in that**
the marker is rod-shaped, and side walls of the recess have, in a longitudinal direction, a multiplicity of projections for clamping the marker.

2. Stent according to Claim 1, in which the marker (3) has a circular cross section and the recess of the stent structure has a wedge shape in order to permit automatic self-centring of the marker when the latter is fitted into the recess.

3. Stent according to Claim 2, in which the wedge-shaped recess emerges into an opening that has a passage directed towards an opposite side of the stent structure.

4. Stent according to Claim 1, in which the recess has a substantially uniform width (B).

5. Stent according to Claim 4, in which the recess has an area of reduced width (b) for clamping the marker (3).

6. Stent according to Claim 4 or 5, in which the marker (3) has a depression for a snap-fit connection of the marker (3) in the recess.

7. Stent according to one of the preceding claims, in which the projections are arranged in an undulating shape.

8. Stent according to one of Claims 1 to 6, in which the projections are arranged as a zigzag profile.

9. Method for improving the radiopacity of a stent, comprising the steps of:
forming a recess in the stent structure;
fitting a marker into the recess; and
filling the recess with a polymer;
**characterized by**
forming the marker in a rod shape, and forming a multiplicity of projections in side walls of the recess in a longitudinal direction for clamping the marker.

## Revendications

1. Stent pour une implantation dans un corps vivant avec un marqueur visible aux rayons X (3, 10, 13) qui est logé dans un évidement (16, 17, 18, 19) de la structure du stent, un volume restant de l'évidement étant rempli d'un polymère (1),
**caractérisé en ce que**
le marqueur est configuré sous forme de tige et **en ce que** des parois latérales de l'évidement comprennent dans une direction longitudinale une pluralité d'avancées pour caler le marqueur.

2. Stent selon la revendication 1, **caractérisé en ce que** le marqueur (3) a une section circulaire et **en ce que** l'évidement de la structure du stent a la forme d'un coin pour permettre le centrage automatique du marqueur lors de son introduction dans l'évidement.

3. Stent selon la revendication 2, l'évidement en forme de coin débouchant dans une ouverture, qui comprend un passage vers un côté opposé de la structure du stent.

4. Stent selon la revendication 1, l'évidement présentant une largeur (B) qui est sensiblement régulière.

5. Stent selon la revendication 4, l'évidement présentant une zone de largeur réduite (b) servant à caler le marqueur (3).

6. Stent selon la revendication 4 ou 5, le marqueur (3) présentant une cavité pour permettre une liaison par enclenchement du marqueur (3) dans l'évidement.

7. Stent selon l'une quelconque des revendications précédentes, les avancées étant disposées en forme d'ondes.

8. Stent selon l'une quelconque des revendications 1 à 6, les avancées étant disposées en tant que profil en zigzag.

9. Procédé pour améliorer la visibilité aux rayons X d'un stent comprenant les étapes suivantes :
réalisation d'un évidement dans la structure du stent ;
mise en place d'un marqueur dans l'évidement et
remplissage de l'évidement avec un polymère,
**caractérisé par**
la configuration du marqueur sous une forme de tige et
par la réalisation dans une direction longitudinale d'une pluralité d'avancées dans des parois latérales de l'évidement pour caler le marqueur.
